(19) Europäisches Patentamt
European Patent Office
Office européen des brevets

(11) Veröffentlichungsnummer: **0 224 256 A1**

# (12) EUROPÄISCHE PATENTANMELDUNG

(21) Anmeldenummer: 86116431.7

(22) Anmeldetag: 26.11.86

(51) Int. Cl.4: C07D 207/273

(30) Priorität: 26.11.85 CH 5036/85

(43) Veröffentlichungstag der Anmeldung: 03.06.87 Patentblatt 87/23

(84) Benannte Vertragsstaaten: AT BE CH DE FR GB IT LI LU NL SE

(71) Anmelder: **LONZA AG**
**Gampel/Wallis(CH)**

(72) Erfinder: **Meul, Thomas, Dr.**
**Balfrinstrasse 21**
**Visp (Kanton Wallis)(CH)**
Erfinder: **McGarrity, Dr.**
**Rathausstrasse 5**
**Visp (Kanton Wallis)(CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40(DE)**

## (54) 4-Alkoxy-2-oxo-pyrrolidin-1-yl-essigsäurealkylester.

(57) Ausgehend von 4-Alkoxy-3-pyrrolin-2-on-1-yl-essigsäureestern wurden durch Hydrierung die neuen 4-Alkoxy-2-oxo-pyrrolidin-1-yl-essigsäurealkylester hergestellt.

Diese Verbindungen stellen wertvolle Zwischenprodukte für die Herstellung des cerebral wirksamen 4-Hydroxy-2-oxo-pyrrolidin-1-yl-acetamides dar.

EP 0 224 256 A1

# 4-Alkoxy-2-oxo-pyrrolidin-1-yl-essigsäurealkylester

Die Erfindung betrifft neue 4-Alkoxy-2-oxo-pyrrolidin-1-yl-essigsäurealkylester sowie ein Verfahren zu deren Herstellung.

Erfindungsgemäss können die neuen Verbindungen als wertvolle stabile Zwischenprodukte für die Synthese des cerebral wirksamen 4-Hydroxy-2-oxo-pyrrolidin-1-yl-acetamids eingesetzt werden.

Es ist aus Pifferi et al., Il Farmaco, Ed.Sc., 1977, 32, 602 bekannt, 4-Hydroxy-2-oxo-pyrrolidin-1-yl-acetamid über 5 Stufen herzustellen. Teure Ausgangsprodukte und eine Gesamtausbeute um 33.8 % machen dieses Verfahren jedoch nicht rentabel.

Es stellte sich folglich die Aufgabe, einen Weg zu finden, der diese Nachteile nicht aufweist.

Auf überraschende Weise konte festgestellt werden, dass mit den neuen Zwischenprodukten der Formel

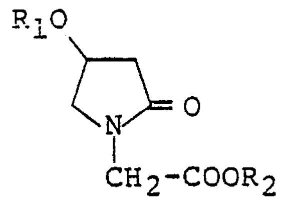

3

worin $R_1$ Alkyl mit 1 bis 2 C-Atomen und $R_2$ Alkyl mit 1 bis 4 C-Atomen bedeuten, die Aufgabe einfach gelöst werden konnte.

Diese neuen Verbindungen, bevorzugt der 4-Methoxy-2-oxo-pyrrolidin-1-yl-essigsäureethylester, sind sehr stabil und können auf einfache Weise nach Patentanspruch 3 hergestellt werden.

So wird zweckmässig von einem 4-Alkoxy-3-pyrrolin-2-on-1-yl-essigsäurealkylester ausgegangen, der seinerseits nach Anweisung des Schweizerischen Patentgesuches 4119/85 auf einfache Weise hergestellt werden kann.

Dieser wird zweckmässig gelöst in einem Lösungsmittel, vorzugsweise in niederen Alkoholen wie Methanol oder Ethanol, in Gegenwart eines üblichen Hydrierungskatalysators mit Wasserstoff hydriert.

Zweckmässig wird als Hydrierungskatalysator Palladium auf einem Trägermaterial, vorteilhaft in einer Konzentration zwischen 5 und 10% auf Kohle, eingesetzt.

Die Einsatzmenge Katalysator wählt man zweckmässig zwischen 1 Mol % und 10 Mol % bezogen auf den eingesetzten 4-Alkoxy-3-pyrrolin-2-on-1-yl-essigsäurealkylester.

Den Druck wählt man zweckmässig zwischen 1 und 20 bar und die Temperatur zweckmässig zwischen 0 und 50°C, vorteilhaft zwischen 20 und 40°C.

Die Aufarbeitung die nach einer Hydrierdauer von 5 bis 20 Stunden erfolgen kann, erfolgt auf übliche Weise z.B. durch Abtrennen des Katalysators, Abdampfen des Lösungsmittels und gegebenenfalls Destillation des Produktes.

Die erfindungsgemässen Verbindungen können so in Ausbeuten über 90% der Theorie und praktisch rein erhalten werden.

Zur Herstellung des cerebral wirksamen 4-Hydroxy-2-oxo-pyrrolidin-1-yl-acetamids können die erfindungsgemässen 4-Alkoxy-2-oxo-pyrrolidin-1-yl-essigsäurealkylester in einer ersten Stufe zum 4-Hydroxy-2-oxo-pyrrolidin-1-yl-essigsäurealkylester umgesetzt werden.

Dies geschieht zweckmässig durch Umsetzen mit Trichlormethylsilan in Gegenwart eines Alkalijodids. Als Alkalijodid wird vorzugsweise das Natriumjodid verwendet.

Zusätzlich arbeitet man vorteilhaft in Gegenwart eines Carbonsäurenitrils, insbesondere eines niederen Carbonsäurenitrils mit 1 bis 6 Kohlenstoffatomen, als Lösungsmittel. Als vorzugsweises Carbonsäurenitril wird Acetonitril verwendet. Zweckmässig wird die Umsetzung bei Rückflusstemperatur des Lösungsmittels durchgeführt.

Nach der für diesen Reaktionstyp üblichen Aufarbeitung und nach einer Filtration über Kieselgel mit polaren, aprotischen Lösungsmitteln, wie halogenierte Kohlenwasserstoffe oder Carbonsäureester, vorzugsweise mit Essigester, kann der 4-Hydroxy-2-oxo-pyrrolidin-1-yl-essigsäurealkylester in guter Ausbeute und Reinheit erhalten werden.

Die anschliessende zweite Stufe, die Amidierung mittels Ammoniak zum 4-Hydroxy-2-oxo-pyrrolidin-1-yl-acetamid wird zweckmässig entsprechend dem bekannten Verfahren von G. Pifferi, M. Pinza, Il Farmaco, Ed. Sc, 32 1977, 602) durchgeführt.

Nach diesem Schritt kann das Endprodukt als weisses Produkt in guter Ausbeute erhalten werden.

## Beispiele

a) Herstellung von 4-Methoxy-2-oxo-pyrrolidin-1-yl-essigsäureethylester

2,00 g (10 mmol) 4-Methoxy-3-pyrrolin-2-on-1-yl-essigsäureethylester wurden in 40 ml Ethanol gelöst, mit 0,20 g Palladium auf Kohle 5% versetzt und 8 Stunden bei einem $H_2$-Druck von 10 bar und bei Raumtemperatur im Autoklaven hydriert. Anschliessend wurde vom Katalysator abfiltriert, das Lösungsmittel im Wasserstrahlvakuum eingedampft und der Rückstand einer Flashdestillation unterworfen. Man erhielt 1,9 g eines farblosen Oels mit einem $Sdp_{0,18}$ :106°C. Dies entsprach einer Ausbeute von 94,5%.

NMR ($CDCl_3$, 300 mHz):
$\delta$ = 4,27 und 3,89 (AB-System, 2H, $J_{AB}$ = 17,7 Hz)
4,19 (q, 2H, $J_{Ester}$ = 7,2 Hz)
4,10 (m, 1H)
3,77 (dd, 1H, J = 10,3 Hz, J = 6,1 Hz)
3,43 (dd, 1H, J = 10,3 Hz, J = 2,6 Hz)
3,34 (s, 3H)
2,67 (dd, 1H, J = 17,4 Hz, J = 6,9 Hz)
2,49 (dd, 1H, J = 17,4 Hz, J = 3,1 Hz)
1,28 (t, 3H, $J_{Ester}$ = 7,2 Hz)
MS (70 eV):
m/z = 202 $M^+$ +1), 169(64), 128(64), 96(100)

b) Herstellung von 4-Hydroxy-2-oxo-pyrrolidin-1-yl-essigsäureethylester

5,00 g (25 mmol) 4-Methoxy-2-oxo-pyrrolidin-1-yl-essigsäureethylester 4,93 g Trichlormethylsilan und 4,95 g Natriumjodid wurden in 75 ml wasserfreiem Acetonitril unter Argon 12 Stunden am Rückfluss erhitzt. Die Reaktionslösung wurde eingedampft, der Rückstand in 200 ml Chloroform aufgenommen und mit gesättigter $NaHCO_3$-Lösung neutralisiert. Anschlissend wurde die $CHCl_3$-Lösung mit gesättigter $Na_2S_2O_3$-Lösung entfärbt. Nach Abtrennen der wässrigen Phase, Trocknen der organischen Phase über $Na_2SO_4$ und Eindampfen erhielt man 5,82 g Rohprodukt. Dieses Rohprodukt wurde mit Essigester über Kieselgel filtriert. Nach Abdampfen des Lösungsmittels erhielt man 2,7 g gelbgefärbtes, öliges Produkt mit einem Gehalt von über 99% (GC). Dies entsprach einer Ausbeute von 58%.

c) Herstellung von 4-Hydroxy-2-oxo-pyrrolidin-1-yl-acetamid

13,7 g (73 mmol ) 4-Hydroxy-2-oxo-pyrrolidin-1-yl-essigethylester wurden gelöst in 500 ml Methanol und bei 0°C mit Ammoniak gesättigt. Danach wurde während einer Stunde bei Raumtemperatur gehalten. Abdampfen des Lösungsmittels unter Vakuum führte zu einem festen Rückstand. Dieser wurde in Methanol kristallisiert.

Es resultierten 7,17 g (62%) des Produktes in Form eines weissen Pulvers. Fp 165 bis 168°C.
IR (Nujol) 3400 $cm^{-1}$, 3300 $cm^{-1}$, 3250 $cm^{-1}$ (OH und NH) 1660 $cm^{-1}$ (C=O).

## Ansprüche

1. 4-Alkoxy-2-oxo-pyrrolidin-1-yl-essigsäure-$C_1$-$C_4$-alkylester

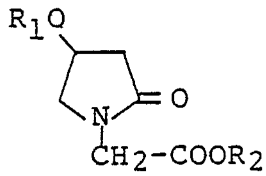

1

worin $R_1$ = $C_1$-$C_2$-alkyl und $R_2$ = $C_1$-$C_4$-alkyl bedeutet.

2. 4-Methoxy-2-oxo-pyrrolidin-1-yl-essigsäureethylester

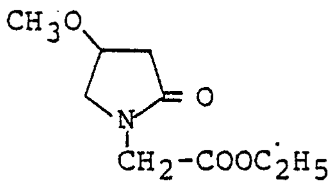

2

3. Verfahren zur Herstellung von Verbindungen gemäss Patentanspruch 1, dadurch gekennzeichnet, dass man einen 4-Alkoxy-3-pyrrolin-2-on-1-yl-essigsäure-$C_1$-$C_4$-alkylester in Gegenwart eines Hydrierungskatalysators mit Wasserstoffhydriert.

4. Verfahren nach Patentanspruch 3, dadurch gekennzeichnet, dass man als Hydrierungskatalysator Palladium, anwendet.

5. Verfahren nach Patentansprüchen 3 und 4, dadurch gekennzeichnet, dass man die Hydrierung bei Drücken zwischen 1 und 20 bar und Temperaturen zwischen 0 und 50°C durchführt.

6. Verwendung der Verbindungen nach Patentanspruch 1 zur Herstellung von 4-Hydroxy-2-oxo-pyrrolidin-1-yl-acetamid, dadurch gekennzeichnet, dass man diese Verbindungen in einer ersten Stufe mit Trichlormethylsilan in Gegenwart eines Alkalijodids und eines niederen Carbonsäurenitrils zum 4-Hydroxy-2-oxo-pyrrolidin-1-yl-essigsäure-$C_1$-$C_4$-alkylester umsetzt und in einer zweiten Stufe diesen durch Amidierung mit Ammoniak zum Endprodukt umsetzt.

7. Verwendung der Verbindung nach Patentanspruch 2 zur Herstellung von 4-Hydroxy-2-oxo-pyrrolidin-1-yl-acetamid, dadurch gekennzeichnet, dass man diese Verbindung in einer ersten Stufe mit Trichlormethylsilan in Gegenwart von Natriumjodid und Acetonitril zum 4-Hydroxy-2-oxo-pyrrolidin-1-yl-essigsäureethylester umsetzt und in einer zweiten Stufe diesen durch Amidierung mittels Ammoniak zum Endprodukt umsetzt.

Europäisches Patentamt

# EUROPÄISCHER RECHERCHENBERICHT

Nummer der Anmeldung

EP 86116431.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | AT - B - 362 782 (I-S-F SOCIETA PER AZIONI)<br>* Seiten 2,3 *<br>-- | 1,3,6 | C 07 D 207/273 |
| A | EP - A2 - 0 154 490 (I.S.F. SOCIETA PER AZIONI)<br>* Patentanspruch 1; Seite 16 *<br>-- | 1,6 | |
| A | CH - A5 - 647 234 (F. HOFFMANN-LA ROCHE & CO. AKTIENGESELLSCHAFT)<br>* Patentansprüche 1,14 *<br>---- | 1,6 | |
| | | | RECHERCHIERTE SACHGEBIETE (Int. Cl.4) |
| | | | C 07 D 207/00 |

Der vorliegende Recherchenbericht wurde für alle Patentansprüche erstellt.

| Recherchenort | Abschlußdatum der Recherche | Prüfer |
|---|---|---|
| WIEN | 03-03-1987 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82